# EUROPEAN PATENT APPLICATION

(11) **EP 4 529 934 A1**
(43) Date of publication of application: **02.04.2025**
(21) Application number: 23200523.1
(22) Date of filing: 28.09.2023
(51) Int. Cl.: A61M 1/00, G16H 20/40

(54) **APPARATUSES AND METHODS FOR NEGATIVE PRESSURE WOUND THERAPY**

(71) Applicant: Mölnlycke Health Care AB, 402 52 Göteborg (SE)
(72) Inventor: BENGTSSON, Erik, 431 41 Mölndal (SE)
(74) Representative: Zacco Sweden AB

(57) **Abstract**

An apparatus and related aspects for providing reduced pressure to a wound site are disclosed. The apparatus comprises a source of negative pressure configured to provide negative pressure via a fluid flow path from an inlet of the source of negative pressure to a wound site. The apparatus further comprises one or more transceivers and one or more antennas, wherein the one or more transceivers is/are configured to transmit data to an external device and to receive data from the external device via the one or more antennas. The apparatus further comprises a user-interface comprising an input device, and control circuitry operatively connected to the source of negative pressure, the user-interface, and to the one or more transceivers. The control circuitry is configured to receive data from the external device, wherein the received data comprises one or more parameter settings for the apparatus. Furthermore, in response to detecting a confirmation act from a user of the apparatus via the input device, the control circuitry is configured to update one or more settings of the apparatus based on the received one or more parameter settings.

## Description

### TECHNICAL FIELD

The herein disclosed technology relates to negative pressure wound therapy (NPWT). In particular, but not exclusively, the disclosed technology relates to an apparatus and a method for providing reduced pressure to a wound site, and thereto related computer program products, computer-readable storage media, and systems.

### BACKGROUND

Negative pressure wound therapy (NPWT) is a technique that promotes healing of e.g. surgical, acute and chronic wounds by the application of a sub-atmospheric pressure ("negative pressure") to the wound, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site. NPWT is applicable to a wide variety of wounds such as open wounds, incisional wounds, skin grafts, and the like.

NPWT apparatuses (may be referred to as pump devices) generally operate based on a set of process parameters that have been carefully considered to ensure the therapy's effectiveness and safety. An example of such process parameters are pressure settings, which define a pressure level or pressure range that the apparatus is to maintain at the wound site. Generally, NPWT is delivered with a pressure setting within the range of -20 mmHg to -300 mmHg, and in particular NPWT apparatuses operate within a subrange of this depending on the type of wound, such as e.g., -50 mmHg to -75 mmHg, -100 mmHg to -150 mm Hg, -115 mmHg to -135 mmHg, or -150 mmHg to -200 mmHg. Other process parameters may include time intervals for different operating modes, duration of therapy, pump motor settings, and so forth.

### SUMMARY

The herein disclosed technology seeks to mitigate, alleviate or eliminate deficiencies and disadvantages in the prior art to address various problems relating to ensuring safe operation of an apparatus providing reduced pressure to a wound site.

It is therefore an object of the herein disclosed technology to provide an apparatus, a method, a computer program product, a computer-readable storage medium, and a system that alleviate all or at least some of the drawbacks of presently known products and methods.

Further, it is an object of the herein disclosed technology to provide an apparatus, a method, a computer program product and a computer-readable storage medium that provide a means for ensuring that the parameters of the apparatus are reliable and accurate in order to ensure adequate function of the apparatus providing reduced pressure to a wound site, and to improve the overall safety of such apparatuses.

Various aspects and embodiments of the herein disclosed technology are defined below and in the accompanying independent and dependent claims.

A first aspect of the disclosed technology comprises an apparatus for providing reduced pressure to a wound site. The apparatus comprises a source of negative pressure configured to provide negative pressure via a fluid flow path from an inlet of the source of negative pressure to a wound site. The apparatus further comprises one or more transceivers and one or more antennas, wherein the one or more transceivers is/are configured to transmit data to an external device and to receive data from the external device via the one or more antennas. The apparatus further comprises a user-interface comprising an input device, and control circuitry operatively connected to the source of negative pressure, the user-interface, and to the one or more transceivers. The control circuitry is configured to receive data from the external device, wherein the received data comprises one or more parameter settings for the apparatus. Furthermore, in response to detecting a confirmation act from a user of the apparatus via the input device, the control circuitry is configured to update one or more settings of the apparatus based on the received one or more parameter settings.

Accordingly, there is provided an NPWT device with an improved functionality, in particular when operating parameters (e.g., pressure settings) for the NPWT device are to be configured or updated. In more detail, the NPWT device is provided with communication capability (e.g., Bluetooth such as Bluetooth Low Energy, BLE) in order to be able to transmit and receive data to and from an external device. This enables a user (e.g., a patient or physician) of the external device (e.g., a smartphone or tablet) to change various operating settings, e.g., therapy related operating parameters such as pressure settings, of the NPWT device. However, it was realized that there is a potential risk that the changed settings are erroneous when the settings are changed via an external device, in particular when there are multiple NPWT devices within a communication range of the external device, such as in a hospital environment. In more detail, the changed operating settings may either be erroneous in themselves or the settings of one NPWT device may be assigned to another NPWT device inadvertently. In either case, erroneous operating settings may reduce the efficiency of the therapy, and in some cases even cause discomfort for the patient. Additionally, NPWT devices whose operating settings can be changed via an external device are also exposed to cyber security related threats where malicious actors may try to change the settings.

Thus, some embodiments herein, incorporate a type of configuration protocol that requires a confirmation act, preferably a physical interaction, to be performed via a user interface (e.g., a button) of the NPWT device before the settings of the NPWT device are updated or otherwise configured. Thereby, the risk of applying erroneous operating settings to an NPWT device is reduced. In particular, the risk of setting operating parameters of a first NPWT device that were intended for a second NPWT device is reduced. The confirmation act also gives the user of the external device a chance to consider the new settings an additional time before actually updating the parameters settings. Accordingly, some embodiments herein improve the reliability and operational safety of NPWT devices.

A second aspect of the disclosed technology comprises a method for controlling an apparatus for providing reduced pressure to a wound site. The method comprises receiving data from an external device via one or more antennas and one or more transceivers of the apparatus, wherein the received data comprises one or more parameter settings for the apparatus. The method further comprises, in response to detecting a confirmation act from a user of the apparatus via an input device of a user-interface of the apparatus, updating one or more settings of the apparatus based on the received one or more parameter settings. With this aspect of the disclosed technology, similar advantages and preferred features are present as in the previously discussed aspect.

A third aspect of the disclosed technology comprises a computer program product comprising instructions which, when the program is executed by a computing device of an apparatus for providing reduced pressure to a wound site, causes the apparatus to carry out the method according to any one of the embodiments disclosed herein. With this aspect of the disclosed technology, similar advantages and preferred features are present as in the previously discussed aspects.

A fourth aspect of the disclosed technology comprises a (non-transitory) computer-readable storage medium comprising instructions which, when executed by a computing device of a vehicle, causes the computing device to carry out the method according to any one of the embodiments disclosed herein. With this aspect of the disclosed technology, similar advantages and preferred features are present as in the previously discussed aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

A fifth aspect of the disclosed technology comprises a wound treatment system comprising an apparatus according to any one of the embodiments disclosed herein, a wound cover for creating a sealed space defined in part by the wound site, and a tubing assembly defining the fluid flow path. With this aspect of the disclosed technology, similar advantages and preferred features are present as in the previously discussed aspects.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

An advantage of some embodiments is the risk of wirelessly setting erroneous operating parameters for an NPWT device when using an external device is reduced, thereby improving the operational safety of NPWT devices.

An advantage of some embodiments is that cyber security related risks for NPWT apparatus with communication capability may be reduced.

An advantage of some embodiments is that the risk of wirelessly updating or configuring a set of operating parameters of the wrong NPWT device in an environment where multiple NPWT devices are in vicinity of each other is reduced.

An advantage of some embodiments is that the reliability of operating parameter update processes for NPWT devices may be more reliable and robust, thereby improving the efficiency of the NPWT treatment.

An advantage of some embodiments is that the risk of patient discomfort due to erroneously set operating settings of the NPWT device may be reduced.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic illustration of a wound treatment system in accordance with some embodiments.
Fig. 3a is a schematic illustration of an apparatus for providing reduced pressure in accordance with some embodiments.
Fig. 3b is a schematic illustration of an apparatus for providing reduced pressure in accordance with some embodiments.
Fig. 4a is a schematic illustration of a plurality of apparatuses for providing reduced pressure to a wound site in accordance with some embodiments.
Fig. 4b is a schematic illustration of a confirmation act in accordance with some embodiments.
Fig. 5a is a schematic illustration of a plurality of apparatuses for providing reduced pressure to a wound site in accordance with some embodiments.
Fig. 5b is a schematic illustration of a confirmation act in accordance with some embodiments.
Fig. 6 is a schematic flowchart representation of a method for controlling an apparatus for providing reduced pressure to a wound site in accordance with some embodiments.

### DETAILED DESCRIPTION

The herein disclosed technology will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

For NPWT apparatuses, several operating parameters (or process parameters) need to be carefully considered to ensure the therapy's effectiveness and safety. These parameters can be adjusted based on the specific needs of the wound and the patient. Some modern NPWT devices have wireless communication capability, and for such NPWT devices it is often possible to set, update, or change at least some of these operating parameters by means of an external device, such as a smart phone or tablet. Using an external handheld device for setting or updating operating parameters of an NPWT device may in some situations have complications. For example, a user of the external device may change an operating parameter by mistake.

Another scenario where complications may arise is in hospital environments where there may be a plurality of patients with NPWT devices in close vicinity to each other. Here, the external device may be in communication range, and even paired with, of a plurality of NPWT devices, and a desired set of process parameters for one patient may be inadvertently assigned to another patient. Another scenario is when a plurality of NPWT devices are arranged on a table or tray prior where the operating settings of each device is to be configured or updated prior to active use. As before, the intended operating settings for one device may be wrongfully assigned to another device. Yet another scenario where complications may arise is when a malicious actor attempts to change various settings of the NPWT device, thereby exposing the NPWT device to cyber security risks.

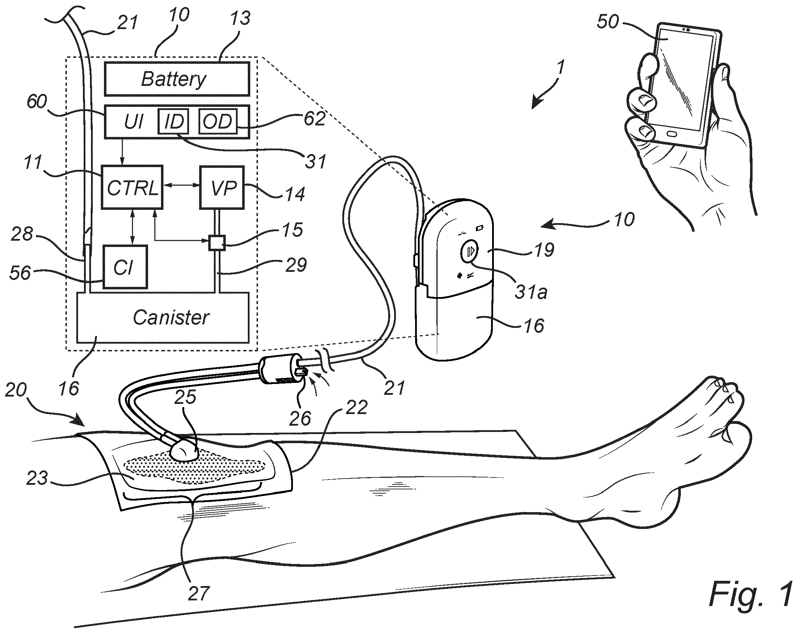

By means of some embodiments disclosed herein it is possible to at least partly mitigate the above-mentioned complications or problems. Turning now to the drawings, and to Fig. 1 in particular, there is schematically illustrated a wound treatment system 1 in accordance with some embodiments. The wound treatment system 1 comprises an apparatus 10 for providing reduced pressure to a tissue site 27 (or otherwise referred to as a wound site 27). The wound treatment system 1 further comprises a wound cover 22 that is adapted to create a sealed space 23 defined in part by a wound surface, such as at the skin of a user/person, at or around a wound of the user/person. Further, the apparatus 10 (may also be referred to as an NPWT device 10) is fluidly connected to the wound cover 22 using e.g. a tubing 21. The tubing 21 may be of any suitable flexible tubing fabricated from elastomeric or polymeric materials. The tubing 21 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing 21 may comprise one or two individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via a fluid flow path from an inlet of the source of negative pressure 14 to the wound dressing 20, or more specifically to provide negative pressure under a wound cover. The source of negative pressure 14 is indicated as "VP" ("Vacuum pump") in the figures. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels. Preferably, the negative pressure level to be achieved is in a range between about -20 mmHg and about -300 mmHg. In some embodiments, a negative pressure range between about -80 mmHg and about -140 mmHg is used. In some embodiments, the negative pressure pump is a diaphragm pump, a peristaltic pump, or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22 or dressing 20. In many cases, the local ambient pressure may also be the atmospheric pressure at which a patient is located. Unless otherwise indicated, values of pressure stated herein are gauge pressures. Similarly, references to increases in negative pressure typically refer to a decrease in absolute pressure, while decreases in negative pressure typically refer to an increase in absolute pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between the canister 16 and the negative pressure source 14 and the dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

The apparatus 10 further comprises control circuitry 11 (may also be referred to as "a control unit", "a controller", or the like) electrically connected to the battery 13 and the source of negative pressure 14. The control circuitry 11 is configured to control operation of the source of negative pressure 14. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein.

Furthermore, in some embodiments, the apparatus 10 comprises one or more pressure sensors 15 arranged in fluid connection with the inlet of the source of negative pressure 14, the canister 16, and/or the sealed space 23. In the depicted embodiment of Fig. 1, the apparatus 10 comprises a pressure sensor 15 arranged in a fluid flow path between the canister 16 and the source of negative pressure 14. However, as readily understood by a person skilled in the art, the pressure sensor 15 may be located at any other suitable location to sense or detect a pressure within the fluid flow path between the source of negative pressure 14 and the wound site. For example, the pressure sensor 15 may be arranged within the canister or under the wound cover 22.

During use of the apparatus 10, the wound cover 22 is arranged at a wound site 27 of the user/patient, forming the sealed space 23. The tubing 21 is provided to fluidly connect the wound cover 22 to the inlet port 28 of the apparatus 10. The apparatus 10 is then activated, e.g. by a user pressing a start/pause button 31a of a user-interface 60 of the apparatus 10. The source of negative pressure 14 is thereby activated. When activated, the source of negative pressure 14 will start to evacuate air through the canister 16, the inlet port 28, the tubing 21 and the sealed space 23 formed by the wound cover 22. Accordingly, a negative pressure will be created within the sealed space 23. In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged between at the outlet port 29 of the canister 16 to ensure that no liquid is allowed to pass to the source of negative pressure 14 from the canister 16.

In some embodiments, the wound treatment system 1 further comprise an air inlet port 26 or a controlled leakage port 26 that provides a small and controlled inflow of air (as indicated by the arrows) under the wound cover 22. The air inlet port 26 may for example be provided via a secondary lumen extending from the connector 25 as schematically illustrated. However, in some embodiments the air inlet port 26 may additionally or alternatively be provided at the connector 25 and/or at any suitable location of the tubing 21.

Accordingly, the wound treatment system 1 may in some embodiments be arranged to ensure that a small leakage is present, ensuring that a flow from the sealed space 23 may be achieved (under the assumption that there is no general blocking e.g. within the tubing 21). Additionally, during use of the wound treatment system 1 some leakage may be expected in relation to the wound cover 22. In some embodiments, the air inlet port 26 includes means for supplying/providing air to the wound site 27 at a predetermined supply rate, the rate being from 2 to 7 ml/min, preferably from 3 to 5 ml/min at a predetermined level of negative pressure. The predetermined level of negative pressure may be from - 80 to -180 mmHg, preferably from -100 to -150 mmHg, more preferably from -110 to -140 mmHg or - 115 to -135 mmHg. The means for providing air to the wound site 27 (i.e. under the wound cover 22) at a predetermined supply rate may for example be in the form of an air filter (not shown).

In some embodiments, the air filter comprises a hydrophobic and porous material, where the size of the pores is within the range of from 2 to 20 µm, preferably in the range of from 5 to 12 µm. The pore size of the filter is measured in a non-compressed state. In some embodiments, the air filter comprises polyethylene or sintered polyethylene.

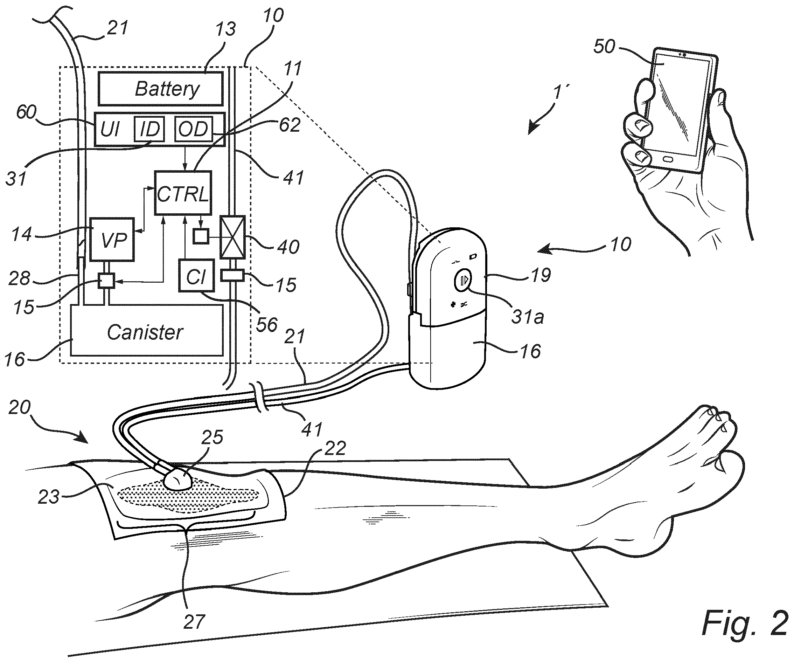

Turning to Fig. 2, a wound treatment system 1' in accordance with some embodiments is schematically illustrated. Many functions, features and components of the wound treatment system 1' depicted in Fig. 2 are the same as or analogous to the wound treatment system 1 depicted in Fig. 1 and will for the sake of brevity and conciseness not be repeated. Instead, the focus will be on the differentiating functions, features, or components between the two wound treatment systems. Thus, as the person skilled in the art readily understands, unless specifically indicated, the foregoing discussion related to the components of the wound treatment system 1 with reference to Fig. 1 is applicable for the corresponding components depicted in Fig. 2 as indicated by the reference numerals.

In general, the wound treatment system 1 depicted in Fig. 1 differs from the wound treatment system 1' depicted in Fig. 2 in that the former is often referred to as a "single-lumen" NPWT system 1 while the latter is often referred to as a "dual-lumen" NPWT system 1'. Accordingly, while the wound treatment system 1 depicted in Fig. 1 has a "controlled inflow of air" (also referred to as a controlled leakage), the wound treatment system 1' depicted in Fig. 2 explicitly controls or regulates the air supply to the wound site 27 by means of the apparatus 10.

Therefore, in contrast to the wound treatment system 1 depicted in Fig. 1, the wound treatment system 1' of Fig. 2 comprises a second fluid flow path that fluidly connects the sealed space 23 created by the wound cover 22 to the ambient atmosphere or a "fluid reservoir" (not shown) via an electronically controllable valve 40. The second fluid flow path (may also be referred to as "air lumen") is defined by a second tubing 41 that may be of any suitable flexible tubing fabricated from elastomeric or polymeric materials. Here, the fluid flow path from the inlet of the source of negative pressure 14 to the wound dressing 20 may be construed as a "first" fluid flow path or "exudate lumen".

Here, the control circuitry 11 is electronically connected to the electronically controllable valve 40, and the control circuitry 11 is further configured to control an operation of the electronically controllable vale 40 so to release negative pressure from the wound site via the second fluid flow path (or "air lumen") 41. In other words, the control circuitry 11 is configured to open the electronically steerable valve 40 so to introduce fluid (e.g. air) to the wound site 27 when there is a sub-atmospheric pressure under the wound cover 22. This operation may also be referred to as "flushing". Since the wound treatment system 1' depicted in Fig. 2 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1'. Therefore, in order to be able to draw desired amounts of wound exudate from the wound site, a fluid, such as e.g. air from the ambient atmosphere, is controllably introduced at defined time intervals or in response to pressure measurements by opening the electronically steerable valve 40. Once enough fluid has been introduced (e.g. in response to a negative pressure within the first and/or second fluid flow path reaching a lower threshold), the control circuitry 11 is configured to close the electronically steerable valve 40 and to activate the source of negative pressure 14.

The ability to controllably "flush" the system 1' by injecting air via an electronically steerable valve 40 may provide the advantage of longer pressure regulation cycles, which reduces the on-time for the source of negative pressure 14 and therefore reduces the energy consumption of the apparatus 10.

Furthermore, regardless of the wound treatment system 1, 1' being single lumen (Fig. 1) or dual lumen (Fig. 2), the apparatus 10 comprises a communication interface 56 having suitable components (e.g. transceivers, antennas, filters, power amplifiers, etc.) with suitable communication protocols (e.g., Wi-Fi, Bluetooth, Bluetooth Low Energy, Zigbee, or the like) in order to establish a connection with an external device 50 and to transmit and receive wireless signals to and from the external device 50. The communication interface 56, indicated as "Cl" is connected to the control circuitry 11, indicated as "CTRL". The communication interface 56 is configured to transmit and receive wireless signals to and from the external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network or cellular or mobile phone communications transceivers. In another example, the communication interface 56 may include a short-range wireless transmitter (e.g., a Bluetooth wireless transmitter, etc.) for communicating via a short-range wireless communication transceiver.

The external device may be an external handheld device 50, such as a mobile phone (e.g. a smart phone) operatively connected to the apparatus 10. The term "operatively connected" is in the context of the present disclosure to be understood as that the "operatively connected" entities or units can transmit and/or receive signals to and/or from each other.

The received signals/data may comprise information about, or otherwise indicate, one or more parameter settings (or parameter setting values) for the apparatus 10. Specifically, the control circuitry 11 is configured to receive data from the external device 50 via the communication interface 56, where the received data comprises one or more parameter settings for the apparatus 10. The parameters settings for the apparatus 10 may comprise various values for one or more operating parameters of the apparatus 10. For example, the parameter settings may comprise one or more of: a set value of negative pressure that the apparatus is to maintain, a negative pressure range that the apparatus is to maintain, one or more pump motor settings, pressure cycle settings (e.g., duration of one pressure cycle), flush settings (e.g., flush intervals, pressure ranges, control valve parameters, etc.), working mode of the apparatus (e.g., silent mode, night mode, or day mode). For example, the currently set negative pressure range of the apparatus 10 may be configured to be between -50 mmHg and -75 mmHg, whereupon the received data comprises a new pressure range setting of the apparatus that is -110 mmHg to -140 mmHg.

Furthermore, the apparatus 10 comprises a user-interface 60, indicated as "Ul" that comprises the input device 31. The input device 31 may be construed as any user-input device by means of which a user of the apparatus 10 can interact. In more detail, the control circuitry 11 is operatively connected (e.g., via a wired connection) to the user-interface 60 and thereby capable of transmitting signals to and receiving signals from the user-interface 60. In some embodiments, the apparatus 10 comprises a housing 19 enclosing the source of negative pressure 14 and the control circuitry 11, where the input device 31 is an integrated part of the housing 19. In some embodiments, the input device comprises a button 31a and/or a touch screen 31b. A "user of the apparatus" 10 is in the present context to be interpreted broadly and may include the patient upon which the NPWT is applied and/or the caregiver.

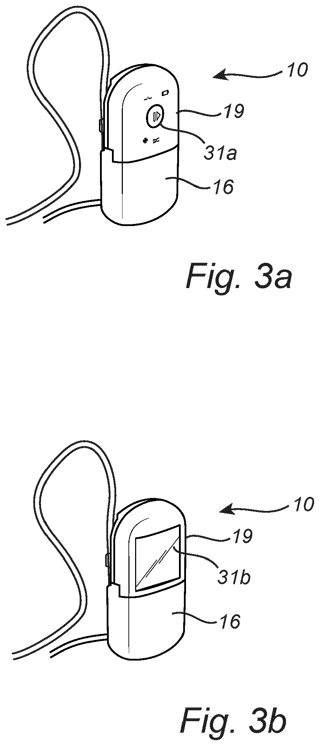

Fig. 3a shows a schematic illustration of an apparatus 10 in accordance with some embodiments. The apparatus 10 comprises a button 31a, where the button 31a may be an integrated part of the housing 19. The button 31a is arranged in relation with the housing 19 such that the button 31 is accessible by a user of the apparatus 10. Furthermore, Fig. 3b shows a schematic illustration of an apparatus 10 in accordance with some embodiments. Here, the apparatus 10 has a user-interface 60 that comprises a touch screen 31b. The touch screen 31b may be an integrated part of the housing 19. The housing 19 and the touch screen 41 may be arranged such that the touch screen 41 is accessible by a user of the apparatus 10.

As mentioned, a potential risk with an apparatus 10 whose settings can be updated via an external device is that the settings of the apparatus 10 may be erroneously or unintentionally changed.

To this end, it is herein proposed to provide an apparatus 10 configured to receive the one or more parameter settings from the external device 50, and in response to detecting a confirmation act from a user of the apparatus 10 via the input device 31, update one or more settings of the apparatus 10 based on the received one or more parameters. Accordingly, the user-interface 60 comprises an input device 31 configured to receive the confirmation act from a user. In some embodiments, the confirmation act may comprise a physical interaction of the user with the input device 31. Thus, before any changes in the settings of such an apparatus 10 are made, the receiving of a confirmation act, such as an acknowledgement act, from the user of the apparatus 10 is needed.

As mentioned, the input device 31 may comprise a button 31a and the control circuitry 11 may be configured such that one or more settings of the apparatus 10 are updated (based on the received data) in response to detecting a confirmation act from a user of the apparatus 10 via the button 31a. The confirmation act may be a physical interaction of the user with the button 31a. For instance, the physical interaction may be pressing on or turning the button 31a. The button 31 may be the same button 31 through which the apparatus 10 is activated initially, e.g. the aforementioned start/pause button 31.

However, the button may be a second button (not shown) that is dedicated for the confirmation act and different from the start/pause button.

As mentioned, the input device 31 may comprise a touch screen 31b and the control circuitry may be configured such that one or more settings of the apparatus 10 is updated (based on the received data) in response to detecting a confirmation act from a user of the apparatus 10 via the touchscreen 31b. The confirmation act may be a physical interaction of the user with the touch screen 31b. The physical interaction may therefore be a physical interaction of the user with the touch screen 31b. The physical interaction may be a swiping action, pressing action or any engagement performed on the touch screen 31b.

Although the illustrated embodiments specify input devices 31 in the form of buttons 31a and touch screens 31b, the input device 31 may comprise any suitable unit that is configured to receive a confirmation act from a user of the apparatus 10. For example, the input device 31 may comprise one or more accelerometers. Accordingly, the confirmation act may comprise moving or tapping/touching the apparatus 10. In other words, the one or more accelerometers may be configured to detect a confirmation act comprising moving or tapping/touching the apparatus 10.

Moreover, in some embodiments, the input device 31 may comprise one or more (wireless) proximity detectors/sensors (e.g., NFC tag, RFID tag, etc.). Accordingly, the confirmation act may comprise moving or otherwise positioning the apparatus 10 in proximity to the external device 50. In other words, the one or more proximity sensors may be configured to detect a confirmation act comprising moving or otherwise positioning the apparatus 10 in proximity to the external device 50. "In proximity to" may in the present context be construed as arranged within 20 cm from each other, arranged within 10 cm from each other, or arranged within 5 cm from each other as defined by the applicable technology or protocol used for the proximity sensor.

Further, in some embodiments, the user-interface 60 further comprises one or more output devices 62. Accordingly, the control circuitry 11 may be further configured to, in response to receiving the data from the external device 50, output a signal to the user of the apparatus 10 via the one or more output devices 62. This implies that a pending update in parameter settings of the apparatus 10 may be communicated to the user by outputting a signal from the apparatus 10. The feature of outputting a signal may be advantageous to identify the apparatus 10 that has received the data from the external device 50 in situations where there is a plurality of apparatuses 10 in close vicinity to each other. In some embodiments, the signal is an audible signal, a visual signal and/or a tactile signal.

For example, the output device 62 may comprise a vibrating unit. The vibrating unit may be configured to cause a vibration of the apparatus 10 upon receiving the data from the external device 50. The control circuitry 11 may be configured to control operation of the vibrating unit and activate the vibrating unit so as to emit a tactile signal to the user of the apparatus 10 upon receiving the data from the external device 50.

Additionally, or alternatively, the output device 62 may comprise a loudspeaker. The loudspeaker may be configured to emit an audible sound to the user of the apparatus 10 upon receiving the data from the external device 50. The control circuitry 11 may be configured to control operation of the loudspeaker and activate the loudspeaker so as to emit an audible signal to the user of the apparatus 10 upon receiving the data from the external device 50.

In some embodiments, the output device 62 may comprise a lighting unit, such as a Light Emitting Diode (LED). The lighting unit may be configured to emit light upon receiving the data from the external device 50. The control circuitry 11 may be configured to control operation of the lighting unit and activate the lighting unit so as to provide a visual signal to the user of the apparatus 10 upon receiving the data from the external device 50.

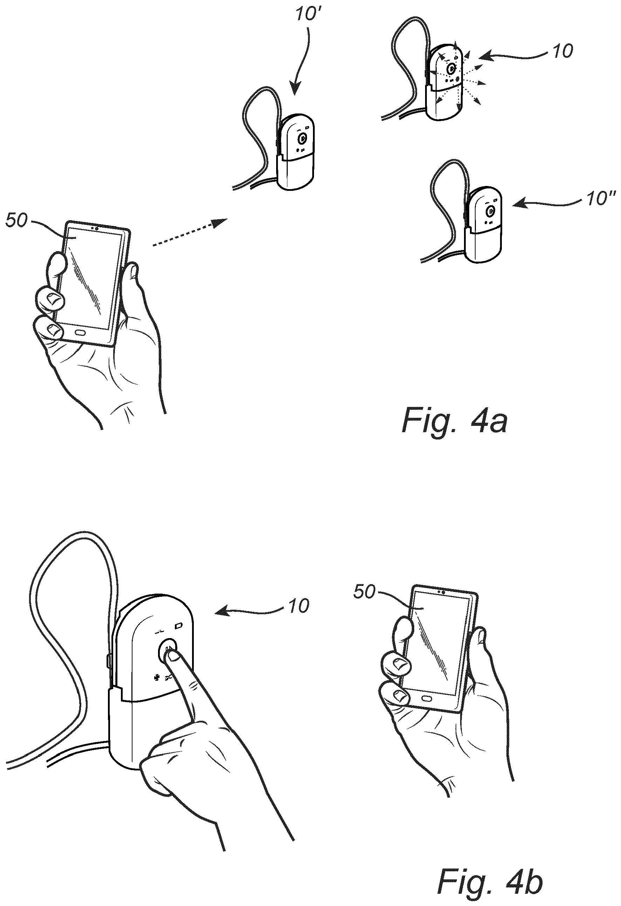

Fig. 4a shows a plurality of apparatuses 10, 10', 10" for providing reduced pressure to a wound site in accordance with some embodiments. Each apparatus 10, 10', 10" comprises a user-interface having an input device with a button 31a and an output device 62 having one or more Light Emitting Diodes (LEDs). The control circuitry 11 of a first apparatus 10 receives data comprising one or more parameter settings for the apparatus 10 from the external device 50. Upon receiving data from the external device 50, the first apparatus 10 outputs a signal in the form of emitting light (e.g., by activating the one or more LEDs of the apparatus 10). The user, in response to detecting the output signal from the apparatus 10, can perform a confirmation act by interacting with the button 31 of the apparatus 10 that is emitting the light and thereby allow the apparatus 10 to update its settings. As mentioned, the apparatus 10 may comprise other input devices, such as e.g., one or more accelerometers and/or one or more proximity sensors (e.g., NFC tags or RFID tags), and the confirmation may then be moving or tapping the apparatus 10 and/or moving the apparatus 10 in proximity to the external device 50, respectively.

Thus, the apparatus 10 assists the user in determining/detecting a pending update of one or more settings of the apparatus 10. Then, in response to detecting the confirmation act in the form of a button press from the user (as shown in Fig. 4b), the control circuitry 11 of the apparatus 10 updates one or more settings of the apparatus 10 based on the parameter settings received from the external device 50. An advantage of having the apparatus 10 output a signal upon receiving data from the external device 50 is that the apparatus 10 that received the data from the external device may be easier to identify by a user handling many apparatuses.

Moreover, by having the apparatus 10 that receives the one or more parameter settings to output a signal the user can manually verify that it was the intended apparatus 10 that received the parameter settings. Moreover, in some instances that external device 50 may broadcast the one or more parameter settings to a plurality of apparatuses 10, 10', 10" whereupon the user can select which of the plurality of apparatuses 10, 10', 10" that should perform the update of one or more settings of the apparatus by interacting the user-interface of that or those apparatuses 10. The output of a signal from the apparatus 10 also indicates to the user that the parameter settings have been received by that specific apparatus 10, thereby providing a guided human-machine interaction.

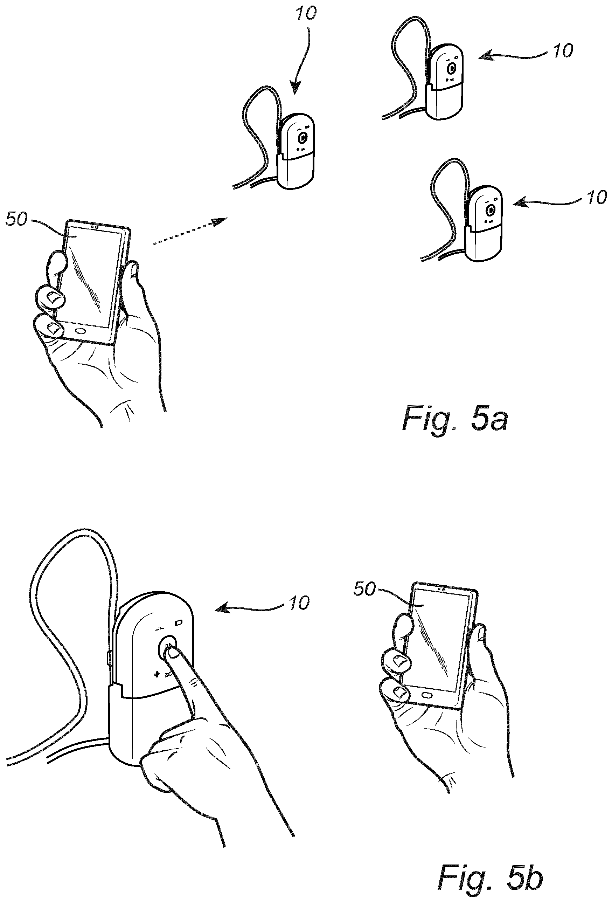

Fig. 5a shows a plurality of apparatuses 10 for providing reduced pressure to a wound site in accordance with some embodiments. Each apparatus 10 comprises a user-interface having an input device with a button 31a. Here, in contrast to the example embodiments depicted in Figs. 4a-b, the apparatuses are not configured to output a signal in response to receiving the data from the external device 50. Instead, the external device 50 that comprises one or more processors and a display apparatus operatively connected to the one or more processors may be configured to instruct the user (i.e. patient or caregiver) to perform the confirmation act by interacting with the input device of the apparatus 10. The external device 50 further comprises a suitable communication interface for transmitting and receiving data to and from the apparatus 10, as readily understood by the skilled person in the art.

In more detail, the one or more processors of the external device 50 may be configured to transmit data comprising one or more parameter settings to the apparatus 10. The one or more processors may be further configured to display via the display apparatus, a graphical user interface (GUI) comprising a graphical representation comprising of a confirmation act on the apparatus 10. Thereby the user may be guided to perform the confirmation act on the apparatus 10 via the GUI of the external device 50. The GUI may for example indicate to the user to press the button on the apparatus 10 as depicted in Fig. 5b. However, the GUI may indicate the user to perform any applicable confirmation act on the apparatus 10.

For example, in some embodiments, the apparatus 10 comprises a matrix barcode (e.g., a OR code) arranged on an outer surface of the housing 19 of the apparatus 10. The matrix barcode is specific for the apparatus 10, meaning that each apparatus 10 has an individual matrix barcode. The confirmation act may accordingly comprise scanning the matrix barcode of the apparatus 10 by means of the external device 50. Here it is assumed that the external device comprises one or more cameras and a suitable application to read the matrix barcode and to transmit a confirmation signal to the apparatus in response to reading the matrix barcode. The confirmation act is detected by the apparatus 10 based on the confirmation signal that is transmitted from the external device 50. Thus, in some embodiments, detecting a confirmation act comprises receiving a confirmation signal from the external apparatus 50, where the confirmation signal is transmitted in response to the matrix barcode of the apparatus 10 being read by the external device 50.

Moreover, the graphical representation may comprise an ID element (e.g., device ID) of the apparatus 10 that received the one or more parameter settings in order to enable the user to identify the correct apparatus 10 for the confirmation act. Moreover, the one or more processors of the external device 50 may be configured to receive an acknowledgement signal from the apparatus 10 that received the one or more parameter settings. The acknowledgement signal may comprise a device ID of the apparatus.

Thereby the external (handheld) device 50, which may be a smartphone or tablet, may provide a GUI that guides the user to confirm the update of one or more settings of the correct apparatus 10, thereby reducing the risk of erroneous parameter setting updates being applied to the apparatus 10.

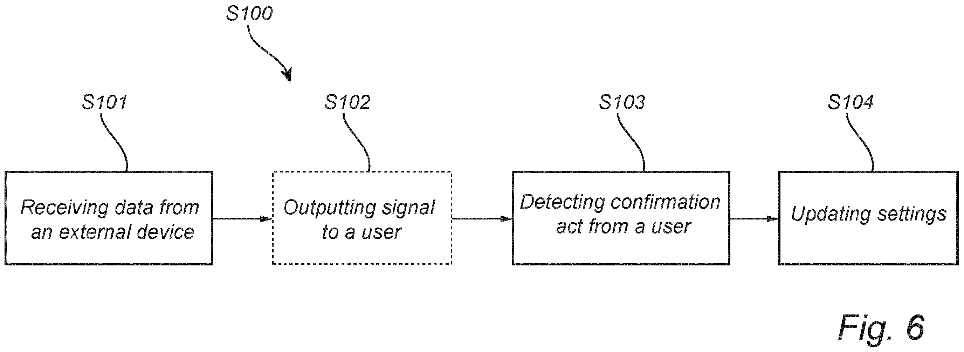

Moving on, Fig. 6 is a schematic flowchart representation of a method S100 for controlling an apparatus for providing reduced pressure to a wound site in accordance with some embodiments.

The method S100 comprises the step of receiving S101 data from an external device via one or more antennas and one or more transceivers of the apparatus. The received data comprises one or more parameter settings for the apparatus. In response to detecting S103 a confirmation act from a user of the apparatus via an input device of a user-interface of the apparatus, the method S100 comprises updating S104 one or more settings of the apparatus based on the received one or more parameter settings.

In some embodiments, the method S100 comprises, in response to receiving the data from the external device, outputting S102 a signal to the user of the apparatus via one or more output devices of the user-interface. In some further embodiments, the signal is an audible signal, a visual signal and/or a tactile signal. However, as mentioned, in some embodiments, the external apparatus is configured to guide the user to perform the confirmation act via a graphical user interface (GUI) of the external device.

In some embodiments, the confirmation act comprises a physical interaction with the input device. Thus, the method S100 may comprise detecting a confirmation act, such as a physical interaction from a user, with the input device or with the apparatus in general.

In some embodiments, the input device comprises a button and/or a touch screen. Thus, the method S100 may comprise detecting a physical interaction from a user with a button and/or a touch screen of the input device and/or with the apparatus in general.

Furthermore, the method S100 may comprise providing an apparatus according to any one of the embodiments disclosed herein.

Executable instructions for performing these functions are, optionally, included in a non-transitory computer-readable storage medium or other computer program product configured for execution by one or more processors of an apparatus for providing reduced pressure to a wound site.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments, there is provided a non-transitory computer-readable storage medium storing one or more programs configured to be executed by one or more processors of an apparatus for providing reduced pressure to a wound site, the one or more programs comprising instructions for performing the method according to any one of the above-discussed embodiments.

The processor(s) 11 (associated with the apparatus 10) may be or include any number of hardware components for conducting data or signal processing or for executing computer code stored in memory. The apparatus 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing reduced pressure to a wound site, the apparatus comprising:
a source of negative pressure (14) configured to provide negative pressure via a fluid flow path from an inlet of the source of negative pressure to a wound site;
one or more transceivers and one or more antennas, wherein the one or more transceivers is/are configured to transmit data to an external device (50) and to receive data from the external device via the one or more antennas;
a user-interface (60) comprising an input device (31);
control circuitry (11) operatively connected to the source of negative pressure (14), the user-interface (60), and to the one or more transceivers;
wherein the control circuitry (11) is configured to:
receive data from the external device (50), wherein the received data comprises one or more parameter settings for the apparatus; and
in response to detecting a confirmation act from a user of the apparatus via the input device (31), update one or more settings of the apparatus (10) based on the received one or more parameter settings.

2. The apparatus (10) according to claim 1, wherein the user-interface (60) comprises one or more output devices (62) and wherein the control circuitry (11) is further configured to, in response to receiving the data from the external device (50), output a signal to the user of the apparatus via the one or more output devices.

3. The apparatus according to claim 2, wherein the signal is an audible signal, a visual signal and/or a tactile signal.

4. The apparatus according to any one of claims 1-3, wherein the confirmation act comprises a physical interaction with the input device.

5. The apparatus according to any one of claims 1-4, wherein the input device comprises a button and/or a touch screen.

6. The apparatus according to any one of claims 1-5, wherein the apparatus comprises a housing enclosing the source of negative pressure and the control circuitry, and wherein the input device is an integrated part of the housing.

7. The apparatus according to any one of claims 1-6, wherein the fluid flow path is a first fluid flow path and wherein the apparatus further comprises an electronically controllable valve configured to release negative pressure from the wound site via a second fluid flow path.

8. A method for controlling an apparatus for providing reduced pressure to a wound site, the method comprising:
receiving data from an external device via one or more antennas and one or more transceivers of the apparatus, wherein the received data comprises one or more parameter settings for the apparatus; and
in response to detecting a confirmation act from a user of the apparatus via an input device of a user-interface of the apparatus, updating one or more settings of the apparatus based on the received one or more parameter settings.

9. The method according to claim 8, wherein the method further comprises, in response to receiving the data from the external device, outputting a signal to the user of the apparatus via one or more output devices of the user-interface.

10. The method according to claim 9, wherein the signal is an audible signal, a visual signal and/or a tactile signal.

11. The method according to any one of claims 8-10, wherein the confirmation act comprises a physical interaction with the input device.

12. The method according to any one of claims 8-11, wherein the apparatus for providing reduced pressure to a wound site is an apparatus according to any one of claims 1-7.

13. A computer program product comprising instructions which, when the program is executed by a computing device of an apparatus for providing reduced pressure to a wound site, causes the apparatus to carry out the method according to any one of claims 8-13.

14. A computer-readable storage medium comprising instructions which, when executed by a computing device of an apparatus for providing reduced pressure to a wound site, causes the apparatus to carry out the method according to any one of claims 8-13.

15. A system comprising:
an apparatus according to any one of claims 1-7;
a wound cover for creating a sealed space defined in part by the wound site;
a tubing assembly defining the fluid flow path.
